(19) Europäisches Patentamt
European Patent Office
Office européen des brevets

(11) Veröffentlichungsnummer: **0 017 238**
**B1**

(12) # EUROPÄISCHE PATENTSCHRIFT

(45) Veröffentlichungstag der Patentschrift: **16.03.83**

(51) Int. Cl.³: **C 07 D 501/36,** C 07 D 501/46, A 61 K 31/545

(21) Anmeldenummer: **80101800.3**

(22) Anmeldetag: **03.04.80**

(54) **Cephemderivate, Verfahren zu ihrer Herstellung und sie enthaltende pharmazeutische Präparate.**

(30) Priorität: **09.04.79 DE 2914327**

(43) Veröffentlichungstag der Anmeldung: **15.10.80 Patentblatt 80/21**

(45) Bekanntmachung des Hinweises auf die Patenterteilung: **16.03.83 Patentblatt 83/11**

(84) Benannte Vertragsstaaten: **AT BE CH DE FR GB IT LI NL**

(56) Entgegenhaltungen:
**DE-A-2 223 375**
**DE-A-2 822 860**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Bormann, Dieter, Dr., Am Schieferberg 37, D-6233 Kelkheim (Taunus) (DE)**
Erfinder: **Dürckheimer, Walter, Dr., Im Lerchenfeld 45, D-6234 Hattersheim am Main (DE)**
Erfinder: **Limbert, Michael, Dr., Frankfurter Strasse 202, D-6233 Kelkheim (Taunus) (DE)**
Erfinder: **Kiesel, Norbert, Dr., Gundelhardtstrasse 2, D-6233 Kelkheim (Taunus) (DE)**
Erfinder: **Seeger, Karl, Dr., Schwalbenweg 9, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Cephemderivate, Verfahren zu ihrer Herstellung und sie enthaltende pharmazeutische Präparate

Gegenstand der Erfindung sind Cephemderivate der allgemeinen Formel I

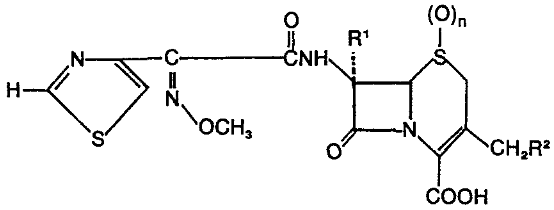

I

in der n für 0 oder 1, $R^1$ für Wasserstoff oder Alkoxy mit 1 bis 4 C-Atomen und $R^2$ für eine Azidogruppe oder einen Rest $-SR^3$ steht, wobei $R^3$ für einen 5gliedrigen Heterocyclus mit 1 bis 4 Stickstoffatomen und gegebenenfalls einem Schwefelatom, einen 5gliedrigen Heterocyclus mit 1 bis 2 Stickstoffatomen und einem Sauerstoffatom oder einen 6gliedrigen Heterocyclus mit 1 bis 3 Stickstoffatomen und gegebenenfalls einem Schwefelatom steht und wobei diese Heterocyclen auch noch an einen Benzolkern ankondensiert oder durch Alkyl mit 1 bis 4 C-Atomen, Trifluormethyl, Carboxymethyl, Hydroxy, Amino, Alkylamino mit 1 bis 4 C-Atomen im Alkylteil, Dialkylamino mit jeweils 1 bis 4 C-Atomen im Alkylteil, Phenyl oder einen 5- oder 6gliedrigen Heterocyclus, der 1 bis 2 Stickstoffatome und gegebenenfalls auch noch ein Schwefel- oder Sauerstoffatom enthalten kann, substituiert sein können, für Alkyl mit 1 bis 4 C-Atomen oder für aliphatisches Acyl mit 1 bis 4 C-Atomen steht und in der die Methoximinogruppe die syn-Position besitzt, sowie deren pharmakologisch verträgliche Salze und Ester.

Gegenstand der Erfindung ist weiterhin ein Verfahren zur Herstellung der Cephemverbindungen der Formel I, das dadurch gekennzeichnet ist, dass man

a) Cephemverbindungen der allgemeinen Formel II

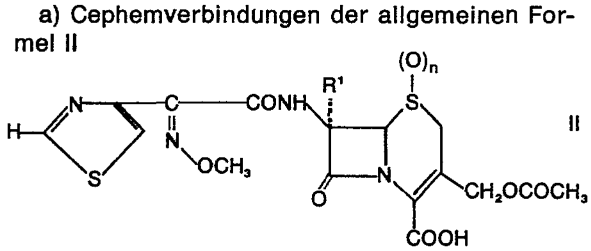

II

in der n und $R^1$ die oben angegebenen Bedeutungen besitzen, in Gegenwart eines Lösungsmittels und einer Base mit einem Azid oder mit Verbindungen der Formel $HSR^3$ umsetzt, wobei $R^3$ die vorstehend angegebenen Bedeutungen besitzt, oder

b) Cephemverbindungen der allgemeinen Formel III in Form ihrer Salze oder Ester

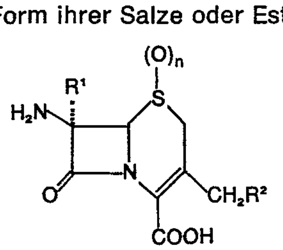

III

in der n, $R^1$ und $R^2$ die angegebenen Bedeutungen besitzen, mit reaktionsfähigen Derivaten der 2-(1.3-Thiazol-4-yl)-2-syn-methoximino-essigsäure zur Reaktion bringt und in den nach a) oder b) hergestellten Verbindungen gewünschtenfalls

α) ein erhaltenes Salz in die freie Carbonsäure überführt und diese gegebenenfalls weiter verestert oder

β) ein erhaltenes Salz direkt in einen Ester überführt oder

γ) einen erhaltenen Ester verseift und gegebenenfalls die erhaltene Carbonsäure in ein Salz überführt oder

δ) eine erhaltene Verbindung der Formel I mit n in der Bedeutung O nachträglich oxydiert oder eine Verbindung der Formel I mit n in der Bedeutung 1 nachträglich reduziert und, falls gewünscht, die erhaltenen Cephemverbindungen nach den angegebenen Schritten α, β oder γ weiter umwandelt.

Aus der DE-A-2 223 375 sind Cephemverbindungen bekannt, die anstelle der 1,3-Thiazol-4-yl-Gruppe einen 1,2-Thiazolylrest (Isothiazolylrest) tragen. Es war nicht zu erwarten, dass sich die erfindungsgemäss erhaltenen Verbindungen gegenüber diesem Stand der Technik durch eine stark überlegene antibakterielle Wirksamkeit auszeichnen würden.

Steht in den Verbindungen der Formel I $R^1$ für Alkoxygruppen mit 1 bis 4 C-Atomen kommen beispielsweise Methoxy, Äthoxy, Isopropoxy oder n-Butoxy in Betracht.

Bevorzugt sind die Verbindungen, in denen $R^1$ für Wasserstoff oder Methoxy steht.

Steht $R^2$ für einen Rest $-SR^3$ und bedeutet $R^3$ aliphatisches Acyl mit 1 bis 4 C-Atomen, so kommen hierfür vorzugsweise der Acetyl- oder Propionylrest in Frage. Bedeutet $R^3$ Alkyl mit 1 bis 4 C-Atomen, so ist darunter geradkettiges oder verzweigtes Alkyl mit 1 bis 4 C-Atomen zu verstehen, wie z.B. Methyl, Äthyl, Isopropyl oder Butyl, vorzugsweise Methyl.

Für den Rest $R^3$ seien beispielsweise folgende grundlegende Ringsysteme genannt:

Thiazolyl, Isothiazolyl, Oxazolyl, Isoxazolyl, Triazolyl, Thiadiazolyl, Oxdiazolyl, Tetrazolyl, Pyridyl, Pyrimidyl, Pyrazinyl, Thiazinyl, Pyridazinyl, Oxazinyl, Imidazolinyl sowie deren benzokondensierte Derivate wie z.B. Benzoxazolyl, Benzthiazolyl, Benzimidazolyl und Indolyl.

Von den vorstehend beispielhaft aufgeführten Ringsystemen kommen vorzugsweise in Betracht 5gliedrige Ringsysteme mit 1 bis 2 Stickstoffatomen und gegebenenfalls einem Sauerstoffatom wie z.B. Oxazolyl, vorzugsweise Oxazol-2-yl, Oxadiazolyl wie z.B. 1.3.4-Oxadiazolyl, Imidazolinyl, vorzugsweise Imidazolin-2-yl und 6gliedrige Ringsysteme mit 1 bis 3, vorzugsweise 1 bis 2, insbesondere einem Stickstoffatom und gegebenenfalls einem Schwefelatom, wie z.B. Pyridyl, wie Pyrid-2-yl, Pyrid-3-yl, Pyrid-4-yl, Pyrimidyl,

vorzugsweise Pyrimid-2-yl und Pyrimid-4-yl, Pyrazinyl oder Pyridazinyl und 5gliedrige Ringsysteme mit 1 bis 4 Stickstoffatomen und gegebenenfalls einem Schwefelatom, insbesondere mit 1 bis 2 Stickstoffatomen und einem Schwefelatomen oder mit 3 oder 4 Stickstoffatomen, insbesondere mit 3 Stickstoffatomen, wie z.B. Thiazolyl, insbesondere Thiazol-2-yl, Thiadiazolyl, insbesondere 1.3.4-Thiadiazol-5-yl und 1.2.4-Thiadiazol-5-yl, Triazolyl, vorzugsweise 4-H-1.2.4-Triazol-3-yl und Tetrazolyl, vorzugsweise 1H-Tetrazol-5-yl.

Der Rest $R^3$ in der Bedeutung von Heterocyclus kann ein- oder mehrfach substituiert sein, beispielsweise durch geradkettige oder verzweigte Alkylgruppen mit 1 bis 4 Kohlenstoffatomen, wie z.B. Methyl, n-Propyl, i-Propyl, n-Butyl, insbesondere Methyl oder durch Hydroxy, Amino, $C_{1-4}$-Alkylamino, $C_{1-4}$-Dialkylamino, Phenyl oder durch 5- oder 6gliedrige Heterocyclen, die 1 bis 2 Stickstoffatome und/oder ein Schwefel- oder Sauerstoffatom im Ring tragen, wie beispielsweise durch Pyrid-2-yl, Pyrid-3-yl, Pyrid-4-yl, Thien-2-yl oder Thien-3-yl.

Als Reste $R^3$ in der Bedeutung von Heterocyclus sind die unsubstituierten und diejenigen bevorzugt, die als Substituenten Amino-, Thienyl oder geradkettiges oder verzweigtes Alkyl mit 1 bis 4 Kohlenstoffatom tragen, wobei das Alkyl seinerseits substituiert sein kann, beispielsweise durch Carboxyl, Halogen, insbesondere Fluor, wie z.B. Methyl, Äthyl, Isopropyl, n-Butyl, Carboxymethyl, Trifluormethyl oder Pentafluoräthyl.

Als pharmakologisch verträgliche Salze der Cephemverbindungen der Formel I kommen anorganische und organische Salze in Betracht, insbesondere die Alkali- und Erdalkalisalze, vorzugsweise die Natrium-, Kalium-, Magnesium- und Calciumsalze, tertiäre oder quartäre Ammoniumsalze, wie z.B. die Triäthylammonium- oder Tetraäthylammoniumsalze oder das Procainsalz. Als pharmakologisch verträgliche Ester seien insbesondere die leicht spaltbaren Ester, wie beispielsweise die Acetoxymethylester, Pivaloyloxymethylester, 1-Acetoxyäthylester sowie der Phthalidester genannt.

Die erfindungsgemäss einzusetzenden Cephemverbindungen der Formel II sind Gegenstand der DE-A-2 822 860.

Die Cephemverbindungen der Formel I können erfindungsgemäss dadurch erhalten werden, dass man die Cephalosporinderivate der Formel II mit Aziden, vorzugsweise Alkaliaziden, insbesondere Natriumazid oder mit Mercaptoverbindungen der Formel $HSR^3$ umsetzt. Die Reaktion wird besonders vorteilhaft in wässriger Lösung oder in Mischungen aus Wasser und mit Wasser mischbaren Lösungsmitteln wie beispielsweise Aceton durchgeführt, wobei durch Zugabe von Basen ein pH von etwa 5.5 bis 7.5 eingestellt wird. Als Basen eigenen sich beispielsweise die Alkalicarbonate, wie z.B. Natriumbicarbonat oder Kaliumbicarbonat, oder die Mischungen aus primärem und sekundärem Alkaliphosphat.

Die Reaktion wird zweckmässigerweise bei erhöhter Temperatur im Bereich von etwa 50 bis 100 °C durchgeführt, wobei der Temperaturbereich von 50 bis 80 °C bevorzugt ist. Die Reaktionszeit hängt von der Temperatur ab, bei der die Umsetzung durchgeführt wird.

Die Mercaptoverbindungen der Formel $HSR^3$, die literaturbekannt sind oder sich nach literaturbekannten Verfahren herstellen lassen, können auch in Form ihrer Salze, insbesondere der Alkalisalze, vorzugsweise der Natrium- und Kakiumsalze, eingesetzt werden, falls diese bei der Herstellung leichter zu handhaben sind. Auch die Verbindungen der Formel II können direkt in Form ihrer Salze, wie z.B. der Alkalisalze, insbesondere der Natrium- oder Kaliumsalze, zum Einsatz kommen.

Die Isolierung der nach dieser Verfahrensvariante erhaltenen Endprodukte gelingt leicht nach im Laborbetrieb üblichen Verfahren. So erhält man beispielsweise die Säurederivate der Formel I, indem man die Cephemsäuren durch Zugabe von Mineralsäuren aus der Reaktionslösung ausfällt. Als Mineralsäuren eigenen sich besonders verdünnte Säuren, wie z.B. verdünnte Salzsäure oder Schwefelsäure.

Die Cephemverbindungen der Formel I fallen bei dieser Verfahrensweise in den meisten Fällen als amorphe Feststoffe oder kristallin an. Sie können alternativ auch durch Extraktion bei etwa pH 2 bis 1 als freie Säuren abgetrennt werden. Als Extraktionsmittel kommen mit Wasser nicht mischbare organische Lösungsmittel in Betracht, wie beispielsweise halogenierte Kohlenwasserstoffe, wie z.B. Methylenchlorid, Ester wie beispielsweise Essigsäureäthylester oder Essigsäure-n-butylester, aber auch Ketone wie z.B. Methylisobutylketon.

Aus den Extrakten werden die entsprechenden Cephemsäuren der Formel I in Substanz gewonnen, beispielsweise durch Einengen, oder gleich in die für eine Darreichung geeignete Form, beispielsweise in ein pharmakologisch verträgliches Salz oder einen Ester überführt.

Die Verbindungen der Formel I lassen sich erfindungsgemäss auch dadurch herstellen, dass man Cephemverbindungen der Formel III in Form ihrer Salze oder Ester mit einem reaktionsfähigen Derivat der 2-(1.3-Thiazol-4-yl)-2-syn-methoximino-essigsäure acyliert. Die Cephemverbindungen der Formel III sind literaturbekannt. Die 2-(1.3-Thiazol-4-yl)-2-syn-methoximino-essigsäure ist Gegenstand der DE-A-2 822 860. Die Umsetzung der 2-(1.3-Thiazol-4-yl)-2-syn-methoximinoessigsäure mit den Cephemverbindungen der Formel III erfolgt in an sich bekannter Weise, beispielsweise durch Aktivierung der Carbonsäuregruppen durch Überführung in eine zur Amidbildung befähigte Gruppierung, vorzugsweise die Überführung in einen Aktivester, beispielsweise den Hydroxybenztriazolester, oder durch Überführung in ein Säurehalogenid, vorzugsweise Säurechlorid, oder durch Überführung in ein symmetrisches oder unsymmetrisches Anhydrid. Alle diese Methoden sind literaturbekannt.

Bei der Aktivierung der Carbonsäure muss darauf geachtet werden, dass durch Wahl möglichst

milder Reaktionsbedingungen, wie sie auch für analoge Reaktionen in der Literatur beschrieben werden, verhindert wird, dass sich das syn-Oxim in das trans-Oximderivat umlagert, das durch die folgende Formel wiedergegeben wird:

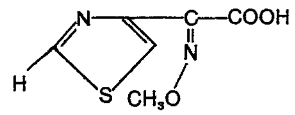

Die Aktivierung soll daher bei möglichst niedrigen Temperaturen im Bereich von etwa −50° bis +50 °C, vorzugsweise von −20° bis +20 °C erfolgen. Zweckmässig ist es weiterhin, stärker saure Reaktionsbedingungen zu vermeiden.

Die Cephemcarbonsäuren der Formel III lassen sich auf verschiedene Weisen in Lösung bringen. Als geeignete Methoden haben sich der Zusatz von Base, aber durch die Überführung in Silylester bewährt. Als Silylierungsmittel eigenen sich die üblichen Reagenzien, insbesondere das Trimethylchlorsilan, das in Gegenwart der stöchiometrischen Menge Base angewendet wird, ganz besonders aber das O,N-Bistrimethylsilylacetamid, das ohne Basenzusatz verwendet werden kann. Zur Erzielung guter Ausbeuten ist es ratsam, das Silylierungsmittel im Verhältnis von etwa 2 Äquivalenten einer Silylverbindung pro Mol Cephemverbindung der Formel III anzuwenden.

Als Basen, die für die Auflösung einer Vielzahl von 7-Amino-△3-cephem-4-carbonsäuren geeignet sind, kommen vor allem organische Basen in Betracht. So haben sich für die Herstellung von Lösungen in organischen Lösungsmitteln insbesondere die tertiären Amine, wie z.B. Triäthylamin, N,N-Dimethylanilin oder N-Methylmorpholin bewährt.

Die Basen werden im allgemeinen in mindestens stöchiometrischer Menge, bezogen auf die gewünschte Umsetzung, zugesetzt. Es empfiehlt sich jedoch, einen Überschuss an Base von beispielsweise etwa 20 bis 80% zu verwenden.

Insbesondere bei gegen Basen empfindlichen Verbindungen der Formel III lässt sich durch kontinuierliche Zugabe der Base je nach Reaktionsverlauf der pH von etwa 4 bis 8, vorzugsweise 6 bis 7 konstant halten.

Die Auflösung der Verbindungen der Formel III kann in einem weiten Temperaturbereich erfolgen. Bei gegen Basen empfindlichen Derivaten empfiehlt es sich jedoch, eine Temperatur von etwa 0 bis 15 °C zu wählen.

Die Acylierung der Cephemverbindungen der Formel III mit der 2-(1.3-Thiazol-4-yl)-2-syn-methoximinoessigsäure kann unter verschiedenen experimentellen Bedingungen durchgeführt werden. So lassen sich die Cephemderivate der Formel III in den unterschiedlichsten Lösungsmitteln acylieren. Besonders geeignet sind beispielsweise organische Lösungsmittel, wie halogenierte Kohlenwasserstoffe, z.B. Methylenchlorid oder Chloroform oder auch tertiäre Amine, wie z.B. Dimethylformamid oder Dimethylacetamid.

Beim Einsatz von Cephemestern der allgemeinen Formel III erfolgt die Umsetzung in organischen Lösungsmitteln, in denen die Ester zumeist gut löslich sind. Als Beispiele für solche Lösungsmittel seien auch hier die halogenierten Kohlenwasserstoffe oder die tertiären Amide genannt.

Als Ester kommen z.B. solche Verbindungen der Formel III in Betracht, in denen in der Estergruppe $-COOR^4$ der Rest $R^4$ für gerades oder verzweigtes Alkyl mit 1 bis 6 C-Atomen stehen kann, wie beispielsweise für Methyl, Äthyl, tert.-Butyl, Isoamyl oder Hexyl, wobei die Alkylgruppe – vorzugsweise in α-Stellung – noch substituiert sein kann beispielsweise durch Trichlormethyl, Acyloxy mit 1 bis 6 C-Atomen, vorzugsweise Acetoxy oder Pivaloyloxy, durch einen oder zwei Phenylreste, die ihrerseits beispielsweise durch Alkoxy mit 1 bis 4 C-Atomen, vorzugsweise Methoxy oder die Nitrogruppe substituiert sein können, oder für die Phthalidgruppe. Als Beispiele für die vorstehen Definitionen seien insbesondere genannt die tert.-Butylester, die Trichloräthyl-, p-Methoxybenzyl-, die Benzhydryl-, Acetoxymethyl-, 1-Acetoxyäthyl-, 1-Propionyloxypropyl, 1-Acetocy-n-butyl-, Pivaloyloxymethylester oder die Phthalidester.

Zu den in Lösung oder gegebenenfalls in Suspension vorliegenden Cephemderivaten der Formel III oder ihren Estern wird die aktivierte 2-(1.3-Thiazol-4-yl)-2-syn-methoximinoessigsäure gelöst oder suspendiert in einem inerten Lösungsmittel oder aber in Substanz hinzugegeben.

Die Reaktionstemperatur hängt weitgehend von der Reaktivität der aktivierten 2-(1.3-Thiazol-4-yl)-2-syn-methoximino-essigsäure ab. Als geeignet hat sich ein Temperaturintervall von etwa −50 bis +50 °C erwiesen. Bevorzugt wird die Umsetzung bei etwa −20 bis +20 °C ausgeführt.

Das aktivierte Säurederivat wird zur Erzielung hoher Ausbeuten in mindestens stöchiometrischer Menge eingesetzt. Ein Überschuss von etwa 5 bis 25% kann sich als zweckmässig erweisen.

Die Isolierung der durch Acylierung erhaltenen Endprodukte erfolgt nach an sich bekannten Methoden, wie sie beispielsweise bereits vorstehend für die durch Austausch der Acetoxygruppe erhaltenen Endprodukte beschrieben wurden. Hat man das symmetrische Anhydrid der 2-(1.3-Thiazol-4-yl)-2-syn-methoximinoessigsäure verwendet, so kann die bei der Acylierung frei gewordene Seitenkettensäure beispielsweise durch Extraktion oder Fällung abgetrennt werden.

Eine alternative Darstellungsweise für Verbindungen der allgemeinen Formel I, in denen n ist, besteht darin, Verbindungen der Formel I mit n = 0 zu oxydieren.

Die Oxydation des Schwefels im Cephemring ist mehrfach beschrieben. Entstehen können dabei α- und β-Oxyde (vgl. z.B. E. Flynn, Cephalosporins and Penicillins, Chemistry and Biology, Academic Press, 1972, S. 135 ff), je nachdem, welches Oxydationsmittel benutzt wird.

Verwendet man beispielsweise Peressigsäure in Eisessig, so erhält man die β-S-Oxyde. Die Reaktionstemperatur ist nicht kritisch, jedoch empfielt es sich, zur Vermeidung von unerwünschten Folgereaktionen, die Oxydation bei Raumtemperatur durchzuführen und das Oxydationsmittel in mindestens stöchiometrischer Menge zuzusetzen, wobei ein Überschuss von 10 bis 100% häufig zweckmässig ist, solange die Temperatur der Reaktion nicht wesentlich gesteigert wird.

Die Isolierung der auf diesem Wege hergestellten Cephem-S-oxyde bereitet keine Schwierigkeiten und kann beispielsweise durch Fällung, z.B. mit unpolaren organischen Lösungsmitteln, und anschliessende Filtration, oder auch durch Extraktion beispielsweise mit n-Butanol, erfolgen.

Falls eine Reduktion der S-Oxide der Verbindungen der allgemeinen Formel I beabsichtigt ist, so kann sie nach literaturbekannten Verfahren vorgenommen werden, wie z.B. durch Behandlung mit Phosphortrihalogeniden, Triphenylphosphin oder Phosphorpentasulfid.

Die Cephemverbindungen der Formel I lassen sich auch durch nachträgliche Veresterung nach literaturbekannten Verfahren in die physiologisch verträglichen Ester der Formel I überführen. So erhält man beispielsweise die Acetoxymethyl- oder Pivaloyloxymethylester durch Umsetzung der Alkalisalze, vorzugsweise der Natriumsalze, oder die Ammoniumsalze, vorzugsweise der Triäthylammoniumsalze, mit den entsprechenden Halogenmethylacylverbindungen, wie beispielsweise Chlormethylacetat, Chlormethylpropionat oder Pivaloylsäurechlormethylester.

Soweit die Ester, insbesondere die physiologisch verträglichen Ester, bereits bei der Acylierung angefallen sind, erübrigt sich eine nachträgliche Veresterung der Carboxylgruppe.

Die bei der erfindungsgemässen Umsetzung direkt angefallenen Ester, wie z.B. p-Methoxybenzyl-, p-Nitrobenzyl-, tert.-Butyl- oder Benzhydrylester können auch in literaturbekannter Weise, beispielsweise durch saure Spaltung oder Hydrierung in die freien Carbonsäuren der Formel I überführt werden.

Die Salze kann man in an sich bekannter Weise durch Umsetzung der Carbonsäure mit geeigneten Basen erhalten.

Die Cephemderivate der Formel I sind wertvolle Antibiotica, die sich überraschend gut zur Bekämpfung gram-positiver und vor allem gram-negativer Infekte eignen und darüber hinaus auch gegen penicillinasebildende Staphylokokken unerwartet gut wirksam sind.

Die erfindungsgemässen Verbindungen können als solche oder zusammen mit den pharmazeutisch üblicherweise eingesetzten Hilfs- und Zusatzstoffen, wie z.B. Traganth, Milchzucker, Talkum, Lösungsmitteln usw. in Form galenischer Zubereitungen, wie z.B. Tabletten, Dragees, Kapseln, Suspensionen, Lösungen usw. peroral, vorzugsweise jedoch parenteral eingesetzt werden, wobei der Wirkstoff in der Regel in einer Menge von etwa 50 bis 1000 mg, vorzugsweise etwa 100 bis 500 mg in einer Verabreichungseinheit enthalten ist.

Für die parenterale Anwendung kommen die üblichen, für den therapeutischen Einsatz geeigneten Lösungsmittel, insbesondere eine Lösung in Wasser in Betracht.

Es ist auch möglich, die erfindungsgemässen Verbindungen mit anderen Wirkstoffen zu kombinieren. So können beispielsweise gleichzeitig andere Antibiotica appliziert werden, wie z.B. solche aus der Reihe der Penicilline, Cephalosporine, Aminoglykosid-Antibiotika oder Verbindungen, die die Symtomatik bakterieller Infektionen beeinflussen, wie z.B. Antipyretika, Antiphlogistica oder Analgetica.

Neben den in den Beispielen beschriebenen Cephemderivaten der Formel I lassen sich beispielsweise auch die folgenden Verbindungen nach den erfindungsgemässen Verfahren herstellen:

7-[2-syn-Methoximino-2-(1.3-thiazol-4-yl)-acetamido]-3-(2-äthyl-1.3.4-thiadiazol-5-yl)-thiomethyl-3-cephem-4-carbonsäure

7-[2-syn-Methoximino-2-(1.3-thiazol-4-yl)-acetamido]-3-(2-methyl-1.3.4-thiadiazol-5-yl)-3-cephem-4-carbonsäure-pivaloyloxymethylester

7-[2-syn-Methoximino-2-(1.3-thiazol-4-yl)-acetamido]-3-(1-methyl-1H-tetrazol-5-yl)-thiomethyl-3-cephem-4-carbonsäure-1-β-S-oxyd

7-[2-syn-Methoximino-2-(1.3-thiazol-4-yl)-acetamido]-3-(2-äthyl-1.3.4-oxdiazol-5-yl)-thiomethyl-3-cephem-4-carbonsäure

7-[2-syn-Methoximino-2-(1.3-thiazol-4-yl)-acetamido]-3-(6-methoxy-pyridazin-3-yl)-thio-methyl-3-cephem-4-carbonsäure

7-[2-syn-Methoximino-2-(1.3-thiazol-4-yl-acetamido]-3-azidomethyl-3-cephem-4-carbon säure-1β-S-oxyd

Beispiel 1:

7-[2-syn-Methoximino-2-(1.3-thiazol-4-yl)-acetamido]-3-[2-thien-2-yl-1,3,4-triazol-5-yl]-thiomethyl-3-cephem-4-carbonsäure

Man löst 1,86 g 2-syn-Methoximino-2-(1.3-thiazol-4-yl)-essigsäure in 67 ccm Dioxan, setzt 1,35 g 1-Hydroxybenztriazol und 2,1 g Dicyclohexylcarboiimid zu und rührt eine Stunde bei Raumtemperatur. Der Niederschlag von Dicyclohexylharnstoff wird abgesaugt. Zu dem Filtrat gibt man eine Lösung von 3,95 g 7-Amino-3-(2-thien-2-yl-1,3,4-triazol-5-yl)-thiomethyl-3-cephem-4-carbonsäure und 1,7 g Natriumbikarbonat in 50 ccm Wasser und rührt 4 Stunden bei Raumtemperatur. Nach Abziehen des Dioxans im Wasserstrahlvakuum stellt man mit 2n-Salzsäure den pH-Wert auf 4, wobei im wesentlichen 1-Hydroxybenztriazol ausfällt. Beim Ansäuern auf pH 2 fällt die Titelverbindung aus, die nacheinander mit je 50 ccm Wasser, Aceton und Äther gewaschen und danach im Vakuum über Phosphorpentoxyd getrocknet wird.

Rf-Wert (Kieselgel/Merck, n-Butanol:Wasser:Eisessig:Äthanol 10:4:3:3 V/V) = 0,4

NMR ($d_6$-DMSO, 60 MHz)

$\delta$ = 3,95 ppm (Singulett, 3H, = N–$OCH_3$)
$\delta$ = 4,25 ppm (AB-Spektrum, 2H, 3–$CH_2$–S–)
$\delta$ = 5,17 ppm (Dublett, 1H, 6–CH)

δ = 5,79 ppm (Quartett 1H, 7–CH)
δ = 7,1–7,6 ppm (Multiplett, 3H, Thienylrest)
δ = 7,94–9,17 ppm (2 Dublett, 2H, Thiazolylrest)
δ = 9,66 ppm (Dublett, 1H, –CONH)

Beispiel 2:
7-[2-syn-Methoximino-2-(1.3-thiazol-4-yl)-acetamido]-3-azidomethyl-3-cephem-4-carbonsäure

Man erhält die Verbindung analog Beispiel 1 in dem man äquivalente Mengen 2-syn-Methoximino-2-(1.3-thiazol-4-yl)-essigsäure als aktivierten Ester mit 7-Amino-3-azido-methyl-3-cephem-4-carbonsäure umsetzt.

Rf-Wert (Trägermaterial u. Lösungsmittel v. Beispiel 1) = 0,3
NMR ($d_6$-DMSO, 60 MHz)

δ = 3,92 ppm (Singulett, 3H, = N–$OCH_3$)
δ = 4,17 ppm (AB-Spektrum, 2H, 3–$CH_2N_3$)
δ = 5,20 ppm (Dublett 1H, 6 CH)
δ = 5,87 ppm (Quartett, 1H, 7 CH)
δ = 7,94 u. 9,17 ppm (2 Dublett, 2H, Thiazolylrest)
δ = 9,73 ppm (Dublett, 1H, –CONH–)

Beispiel 3:
7-[2-syn-Methoximino-2-(1.3-thiazol-4-yl)-acetamido]-3-(1-methyl-1H-tetrazol-5-yl)-thiomethyl-3-cephem-4-carbonsäure

Man löst 4,4 g 7-[2-syn-Methoximino-2-(1.3-thiazol-4-yl)-acetamido]-cephalosporansäure in 10 ccm 1n-Natronlauge und 50 ccm Wasser, setzt 1,75 g 2-Mercapto-1-methyl-1H-tetrazol (Natriumsalz) gelöst in 25 ccm Wasser zu und rührt unter Stickstoff 6 Stunden bei 60 °C. Nach Abkühlen stellt man unter Eiskühlung mit 2n-Salzsäure den pH-Wert auf 2. Die Titelverbindung fällt aus, wird mit Wasser gewaschen und danach im Vakuum über Phosphorpentoxid getrocknet.

Rf-Wert (Kieselgel/Merck, n-BuOH:Eisessig: Wasser = 3:1:1 V/V) = 0.60
NMR ($d_6$-DMSO·60MHz)

δ = 4,0 ppm (Singulett, 3H, = N–$OCH_3$)
δ = 4,25 ppm (Ab-Spektrum, 2H, 3–$CH_2$–S)
δ = 5,10 ppm (Dublett 1H, 6–CH–)
δ = 5,76 ppm (Quartett, 1H, 7–CH–)
δ = 7,91 u. 9,12 ppm (2 Dublett, 2H, Thiazolylrest)
δ = 9,59 ppm (Dublett, 1H, –CONH–)

Beispiel 4:
7-[2-syn-Methoximino-2-(1.3-thiazol-4-yl)-acetamido]-3-(2-methyl-1,3,4-thiadiazol-5-yl)-thiomethyl-3-cephem-4-carbonsäure

4,4 g 7-[2-syn-Methoximino-2-(1.3-thiazol-4-yl)-acetamido]-cephalosporansäure und 1,3 g 5-Mercapto-2-methyl-1,3,4-thiadiazol werden in 75 ccm Wasser suspendiert, unter Rühren 20 ccm 1n-Natronlauge vorsichtig zugegeben und 2 Stunden auf 80° erhitzt. Man kühlt mit Eis, säuert mit Salzsäure auf pH 2 an und saugt die ausgefallene Titelverbindung ab. Nach gründlichem Auswaschen mit Wasser wird über Phosphorpentoxyd im Vakuum getrocknet.

Rf (DC, Trägermaterial und Laufmittel von Beispiel 3) = 0.60
NMR ($d_6$-DMSO, 60 MHz)

δ = 2,73 ppm (Singulett, 3H, –$CH_3$)
δ = 3,96 ppm (Singulett, 3H, –$OCH_3$)
δ = 4,40 ppm (AB-Spektrum, 2H, 3–$CH_2$S–)
δ = 5,21 ppm (Dublett, 1H, 6–CH–)
δ = 5,83 ppm (Quartett, 1H, 7–CH–)
δ = 7,94 u. 9,20 ppm (3 Dublett, 2H, Thiazolylrest)
δ = 9,66 ppm (Dublett, 1H, –CINH–)

Beispiel 5:
7-[2-syn-Methoximino-2-(1.3-thiazol-4-yl)-acetamido]-3-(1H-1-methyl-2-trifluormethyl-1,3,4-triazol-5-yl)-thiomethyl-3-cephem-4-carbonsäure

4,4 g 7-[2-syn-Methoximino-2-(1.3-thiazol-4-yl)-acetamido]-cephalosporansäure werden in 50 ccm Wasser suspendiert und unter Rühren 10 ccm 1n-Natronlauge vorsichtig zugegeben. Zu der Lösung setzt man 1,8 g 5-Mercapto-1H-1-methyl-2-trifluormethyl-1,3,4-triazol, gelöst in 15 ccm Wasser und 10 ccm 1n-Natronlauge, und rührt unter Stickstoff 10 Stunden bei 50 °C. Anschliessend stellt man unter Eiskühlung den pH-Wert auf 2, saugt die ausgefallene Titelverbindung ab, wäscht gründlich mit Wasser und trocknet im Vakuum über Phosphorpentoxid.

Rf-Wert (DC, Trägermaterial und Laufmittel von Beispiel 1) = 0.25
NMR ($d_6$-DMSO, 60 MHz)

δ = 3,94 ppm (Singulett, 3H, = N–$CH_3$)
δ = 4,12 ppm (AB-Spektrum 2H, 3–$CH_2$–S–)
δ = 5,13 ppm (Dublett, 1H, 6–CH)
δ = 5,81 ppm (Quartett, 1H, 7–CH)
δ = 7,92 und 9,17 ppm (2 Dublett, 2H, Thiazolylrest)

Beispiel 6:
7-[2-syn-Methoximino-2-(1.3-thiazol-4-yl)-acetamido]-3-(4,6-diamino-pyrimidin-2-yl)-thiomethyl-3-cephem-4-carbonsäure

Durch Reaktion äquivalenter Mengen 7-[2-syn-Methoximino-2-(1.3-thiazol-4-yl)-acetamido]-cephalosporansäure und 4,6-Diamino-2-mercepto-pyrimidin erhält man analog Beispiel 3 die Titelverbindung.

Rf-Wert (DC-Laufmittel von Beispiel 1) = 0.24
NMR ($d_6$-DMSO, 60 MHz)

δ = 3,94 ppm (Singulett, 3H, = N–$OCH_3$)
δ = 5,17 ppm (Dublett, 1H, 6–CH)
δ = 5,79 ppm (Quartett, 1H, 7–CH)
δ = 6,15 ppm (Singulett, 1H, Pyrimidinrest)
δ = 6,83 ppm (Singulett, 4H, Diamino-)
δ = 7,92 und 9,17 ppm (2 Dublett, 2H, Thiazolylrest)
δ = 9,63 ppm (Dublett, 1H, –CONH–)

Beispiel 7:

7-[2-syn-Methoximino-2-(1.3-thiazol-4-yl)-acetamido-3-(5-carboxymethyl-4-methyl-thiazol-2-yl)-thiomethyl-3-cephem-4-carbonsäure

Man erhält die Titelverbindung analog Beispiel 3 in dem man äquivalente Mengen 7-[2-syn-Methoximino-2-(1.3-thiazol-4-yl)-acetamido]-cephalosporansäure und 5-Carboxymethyl-2-mercapto-4-methyl-thiazol umsetzt.

Rf-Wert: (Kieselgel/Merck, Aceton:Eisessig: 20:1, V/V) = 0,32

NMR ($d_6$-DMSO, 60 MHz)

δ = 2,20 ppm (Singulett, 3H, 4–$CH_3$)
δ = 3,89 ppm (Singulett, 3H, = N–$OCH_3$)
δ = 5,10 ppm (Dublett, 1H, 6–CH–)
δ = 7,88 u. 9,10 ppm (2 Dublett, 2H, Thiazolylrest)
δ = 9,58 ppm (Dublett, 1H, –CO–NH–)

Beispiel 8:

7-[2-syn-Methoximino-2-(1.3-thiazol-4-yl)-acetamido]-3-(1-methyl-1H-tetrazol-5-yl)-thiomethyl-3-cephem-4-carbonsäure

Zu einer Suspension von 7,4 g 2-syn-Methoximino-2-(1.3-thiazol-4-yl)-essigsäure in 75 ml Methylenchlorid werden 3,6 ml Dimethylacetamid unter Feuchtigkeitsausschluss bei −10° hinzugefügt und anschliessend bei −10° 25 ml einer 21,3prozentigen Phosgenlösung in Toluol innerhalb von 15 min zugetropft. Die Reaktionsmischung rührt man 2 Stunden, wobei sich das 2-syn-Methoimino-2-(1.3-thiazol-4-yl)-essigsäurechlorid bildet. Zu der Reaktionsmischung wird anschliessend eine Lösung von 13.1 g 7-Amino-3-(1-methyl-1-H-tetrazol-5-yl)-thiomethyl-3-cephem-4-carbonsäure in 200 ml Methylenchlorid, 10.3 ml Triäthylamin und 6.8 g Pyrolidon bei −10° zugetropft. Die Reaktionsmischung wird 2 Stunden bei −5° gerührt, anschliessend die organische Phase mit Wasser versetzt, auf pH 7 gestellt und die wässrige Phase abgetrennt. Die wässrige Phase wird zuerst auf pH 3,5 gestellt, der dabei anfallende Niederschlag wird abgetrennt und anschliessend auf pH1 angesäuert. Die Titelverbindung fällt als amorpher Feststoff aus, der isoliert, mit Wasser gewaschen und im Hochvakuum über $P_2O_5$ getrocknet wird. Die erhaltene Verbindung ist nach Dünnschichtchromatogramm, NMR und IR identisch mit der in Beispiel 3 erhaltenen Verbindung.

Beispiel 9:

7-[2-syn-Methoximino-2-(1.3-thiazol-4-yl)-acetamido]-3-(2-methyl-1.3.4-oxdiazol-5-yl)-thiomethyl-3-cephem-4-carbonsäure

Zu einer Lösung von 1,32 g 7-(2-syn-Methoximino-2-(1.3-thiazol-4-yl)-acetamido]-cephalosporansäure und 0,75 g Natriumbicarbonat in einer Mischung aus 30 ml Wasser und 10 ml Aceton werden 580 mg 2-Mercapto-5-methyl-1.3.4-oxdiazol gegeben und die Reaktionsmischung 6 Stunden auf 65° erwärmt. Anschliessend wird das Aceton im Vakuum entfernt, die wässrige Lösung auf pH 4 angesäuert und mit Äther das überschüssige Oxdiazol entfernt. Die wässrige Phase wird anschliessend auf pH 1.5 angesäuert, wobei die Titelverbindung in Form cremefarbener Kristalle ausfällt, die mit Wasser und Äther gewaschen und anschliessend über Phosphorpentoxid im Vakuum getrocknet werden.

Rf-Wert (Kieselgel/Merck, n-BuOH:Wasser:Eisessig:Äthanol 10:4:3:3 V/V) = 0,34

NMR: ($CF_3COOD$ 60 MHz)

δ = 2,88 ppm (Singulett, 3H, $CH_3$-Oxdiazolyl)
δ = 3,88 ppm (verbreit. Singulett, 2H, S–$CH_2$)
δ = 4,45 ppm (Singulett, 3H, =N–$OCH_3$)*
δ = 4,55 ppm (AB, 2H, 3–$CH_2$–S–)*
δ = 5,33 ppm (Dublett, 1H, 6–CH)
δ = 6,10 ppm (Dublett, 1H, 7–CH)
δ = 8,66 ppm (Dublett, 1H, 2.Thiazol-H in $CF_3COOD$ nicht messbar)

Thiazol-H in $d_6$-DMSO, 60 MHz:7,90 und 9,13 (2 Dublett, 2H) * Gesamtintregration 5H

Beispiel 10:

7-[2-syn-Methoximino-2-(1.3-thiazol-4-yl)-acetamido]-3-(benzthiazol-2-yl)-thiomethyl-3-cephem-4-carbonsäure

1,32 g 7-[2-syn-Methoximino-2-(1.3-thiazol-4-yl)-acetamido]-cephalosporansäure werden in 30 ml Wasser und 10 ml Aceton mit 0,75 g Natriumbicarbonat gelöst und unter Rühren mit 0,85 g 2-Mercaptobenzthiazol versetzt. Die Reaktionsmischung wird 6 Stunden bei 65° gerührt, anschliessend gekühlt, auf pH 1 angesäuert und mit Methylenchlorid mehrmals extrahiert. Die vereinten Extrakte werden mit Natriumsulfat getrocknet, eingeengt und der verbleibende Rückstand mit Äther digeriert. Es bleibt ein cremefarbener Feststoff, der isoliert, mit Äther nachgewaschen und anschliessend im Hochvakuum über $P_2O_5$ getrocknet wurde.

Rf-Wert (DC, Trägermaterial und Laufmittel von Beispiel 1) = 0,53

NMR: ($d_6$-DMSO, 60 MHz)

δ = 3,92 ppm (Singulett, 3H, =$NOCH_3$)
δ = 4,53 ppm (AB-Spektrum, 2H, –$CH_2$–S)
δ = 5,12 ppm (Dublett, 1H, 6–CH)
δ = 5,76 ppm (Quartett, 1H, 7–CH)
δ = 7,2–8,1 (Multiplett, 4H, Benzthiazol und Dublett 7,90 1H, Thiazolylrest)
δ = 9,13 ppm (Dublett, 1H, Thiazolylrest)
δ = 9,65 ppm (Dublett, 1H, CONH)

Beispiel 11:

7-[2-syn-Methoximino-2-(1.3-thiazol-4-yl)-acetamido]-3-(6-hydroxy-pyridazin-3-yl)-thiomethyl-3-cephem-4-carbonsäure

In der in Beispiel 10 angegebenen Weise erhält man bei der Umsetzung von 7-[2-syn-Methoximino-2-(1.3-thiazol-4-yl)-acetamido]-cephalosporansäure mit 6-Hydroxy-3-mercaptopyridazin bei der Extraktion mit Essigester die Titelverbindung, die beim Digerieren mit Äther als beigefarbener Feststoff isoliert wird.

Rf-Wert (Kieselgel/Merck, Laufmittel von Beispiel 1) = 0,35
NMR: ($d_6$-DMSO, 60 MHz)

δ = 3,90 ppm (Singulett, 3H, $=NOCH_3$)
δ = 4,50 ppm (AB-Spektrum, 2H, $-CH_2-S$)
δ = 5,12 ppm (Dublett, 1H, 6–CH)
δ = 5,70 ppm (Quartett, 1H, 7–CH)
δ = 6,88 ppm und 7,65 ppm (2 Dubletts, je 1H, Pyridazinyl)
δ = 7,90 ppm und 9,05 ppm (2 Dubletts, je 1H, Thiazolyl)
δ = 9,45 ppm (Dublett, 1H, CONH)

**Patentansprüche für den Vertragsstaat:** AT

1. Verfahren zur Herstellung von Cephemverbindungen der allgemeinen Formel I

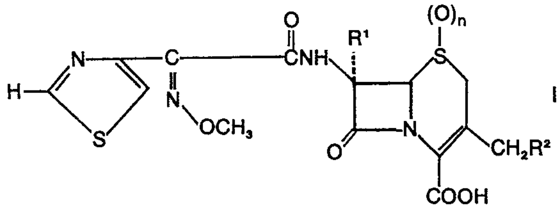

I

in der n für 0 oder 1, $R^1$ für Wasserstoff oder Alkoxy mit 1 bis 4 C-Atomen und $R^2$ für eine Azidogruppe oder einen Rest $-SR^3$ steht, wobei $R^3$ für einen 5gliedrigen Heterocyclus mit 1 bis 4 Stickstoffatomen und gegebenenfalls einem Schwefelatom, einen 5gliedrigen Heterocyclus mit 1 bis 2 Stickstoffatomen und einem Sauerstoffatom oder einen 6gliedrigen Heterocyclus mit 1 bis 3 Stickstoffatomen und gegebenenfalls einem Schwefelatom steht und wobei diese Heterocyclen auch noch an einen Benzolkern ankondensiert oder durch Alkyl mit 1 bis 4 C-Atomen, Trifluormethyl, Carboxymethyl, Hydroxy, Amino, Alkylamino mit 1 bis 4 C-Atomen im Alkylteil, Dialkylamino mit jeweils 1 bis 4 C-Atomen im Alkylteil, Phenyl oder einen 5- oder 6gliedrigen Heterocyclus, der 1 bis 2 Stickstoffatome und gegebenenfalls auch noch ein Schwefel- oder Sauerstoffatom enthalten kann, substituiert sein können, für Alkyl mit 1 bis 4 C-Atomen oder für aliphatisches Acyl mit 1 bis 4 C-Atomen steht und in der die Methoximinogruppe die syn-Position besitzt, sowie deren pharmakologisch verträgliche Salze und Ester, dadurch gekennzeichnet, dass man

a) Cephemverbindungen der allgemeinen Formel II

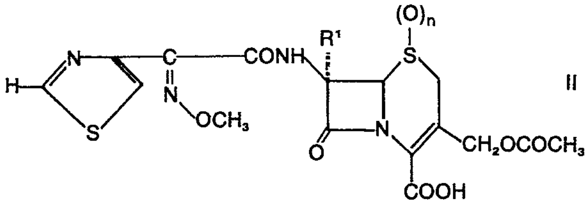

II

in der $R^1$ und n die oben angegebenen Bedeutungen besitzen, in Gegenwart eines Lösungsmittels und einer Base mit einem Azid oder mit Verbindungen der Formel $HSR^3$ umsetzt, wobei $R^3$ die vorstehend angegebenen Bedeutungen besitzt oder

b) Cephemverbindungen der allgemeinen Formel III in Form ihrer Salze oder Ester

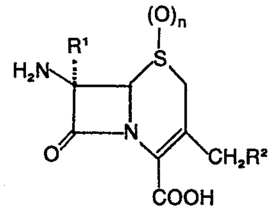

III

in der n, $R^1$ und $R^2$ die oben angegebenen Bedeutungen besitzen, mit reaktionsfähigen Derivaten der 2-(1.3-Thiazol-4-yl)-2-syn-methoximinoessigsäure zur Reaktion bringt und in den nach a) oder b) hergestellten Verbindungen gewünschtenfalls

α) ein erhaltenes Salz in die freie Carbonsäure überführt und diese gegebenenfalls weiter verestert oder

β) ein erhaltenes Salz direkt in einen Ester überführt oder

γ) einen erhaltenen Ester verseift und gegebenenfalls die erhaltene Cephemsäure in ein Salz überführt oder

δ) eine erhaltene Verbindung der Formel I mit n in der Bedeutung 0 nachträglich oxydiert oder eine Verbindung der Formel I mit n in der Bedeutung 1 nachträglich reduziert und, falls gewünscht, die erhaltenen Cephemverbindungen nach den angegebenen Schritten α, β oder γ weiter umwandelt.

2. Verfahren nach Anspruch 1, dadurch gekennzeichnet, dass man die Verbindungen der Formel II und der Formel $HSR^3$ in Form ihrer Salze einsetzt.

**Patentansprüche für die Vertragsstaaten:** BE, CH, DE, FR, GB, IT, LI, NL

1. Cephemverbindungen der allgemeinen Formel I

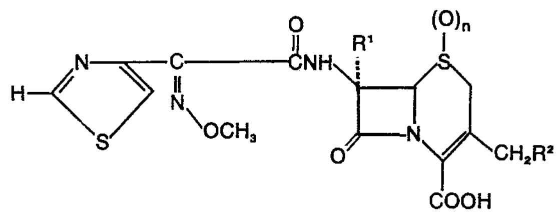

I

in der n für 0 oder 1, $R^1$ für Wasserstoff oder Alkoxy mit 1 bis 4 C-Atomen und $R^2$ für eine Azidogruppe oder einen Rest $-SR^3$ steht, wobei $R^3$ für einen 5gliedrigen Heterocyclus mit 1 bis 4 Stickstoffatomen und gegebenenfalls einem Schwefelatom, einen 5gliedrigen Heterocyclus mit 1 bis 2 Stickstoffatomen und einem Sauerstoffatom oder einen 6gliedrigen Heterocyclus mit 1 bis 3 Stickstoffatomen und gegebenenfalls einem Schwefelatom steht und wobei diese Heterocyclen auch noch an einen Benzolkern ankondensiert oder durch Alkyl mit 1 bis 4 C-Atomen, Trifluormethyl, Carboxymethyl, Hydroxy, Amino, Alkylamino mit 1 bis 4 C-Atomen im Alkylteil, Dialkylamino mit jeweils 1 bis 4 C-Atomen im Alkylteil, Phenyl oder einen 5- oder 6gliedrigen Heterocyclus, der 1 bis 2 Stickstoffatome und gegebenenfalls auch noch ein Schwefel- oder Sauerstoffatom enthalten kann, substituiert sein können, für Alkyl mit 1 bis 4 C-Atomen oder für aliphatisches Acyl mit 1 bis 4 C-Atomen steht und in der die Methoximinogruppe die syn-Position besitzt, sowie deren pharmakologisch verträgliche Salze und Ester.

2. Verfahren zur Herstellung von Cephemverbindungen der allgemeinen Formel I, dadurch gekennzeichnet, dass man

a) Cephemverbindungen der allgemeinen Formel II

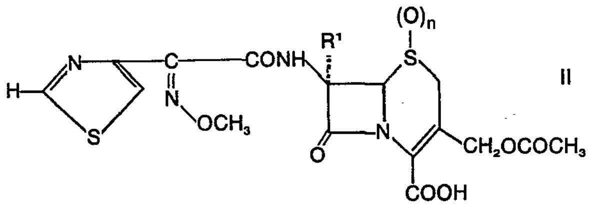

II

in der $R^1$ und n die oben angegebenen Bedeutungen besitzen, in Gegenwart eines Lösungsmittels und einer Base mit einem Azid oder mit Verbindungen der Formel $HSR^3$ umsetzt, wobei $R^3$ die vorstehend angegebenen Bedeutungen besitzt oder

b) Cephemverbindungen der allgemeinen Formel III in Form ihrer Salze oder Ester

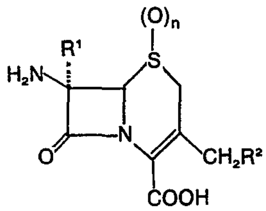

III

in der n, $R^1$ und $R^2$ die oben angegebenen Bedeutungen besitzen, mit reaktionsfähigen Derivaten der 2-(1.3-Thiazol-4-yl)-2-syn-methoximinoessigsäure zur Reaktion bringt und in den nach a) oder b) hergestellten Verbindungen gewünschtenfalls

α) ein erhaltenes Salz in die freie Carbonsäure überführt und diese gegebenenfalls weiter verestert oder

β) ein erhaltenes Salz direkt in einen Ester überführt oder

γ) einen erhaltenen Ester verseift und gegebenenfalls die erhaltene Cephemsäure in ein Salz überführt oder

δ) eine erhaltene Verbindung der Formel I mit n in der Bedeutung 0 nachträglich oxydiert oder eine Verbindung der Formel I mit n in der Bedeutung 1 nachträglich reduziert und, falls gewünscht, die erhaltenen Cephemverbindungen nach den angegebenen Schritten α,β oder γ weiter umwandelt.

3. Verfahren nach Anspruch 2, dadurch gekennzeichnet, dass man die Verbindungen der Formel II und der Formel $HSR^3$ in Form ihrer Salze einsetzt.

4. Gegen bakterielle Infektionen wirksame pharmazeutische Präparate, gekennzeichnet durch einen Gehalt an Cephemderivaten der allgemeinen Formel I.

**Revendications pour les Etats contractants:** BE, CH, DE, FR, GB, IT, LI, NL

1. Composés de céphème répondant à la formule générale I

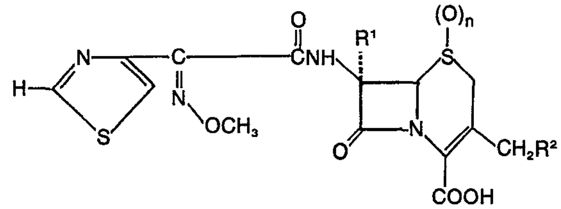

I

dans laquelle n est 0 ou 1, $R^1$ représente l'hydrogène ou un groupe alcoxy ayant de 1 à 4 atomes de carbone et $R^2$ représente un groupe azido ou $-SR^3$, où $R^3$ représente un hétérocycle à 5 chaînons ayant de 1 à 4 atomes d'azote et éventuellement un atome de soufre, un hétérocycle à 5 chaînons ayant de 1 à 2 atomes d'azote et un atome d'oxygène ou un hétérocycle à 6 chaînons ayant de 1 à 3 atomes d'azote et éventuellement un atome de soufre et où ces hétérocycles peuvent également être encore condensés sur un noyau benzène ou peuvent être substitués par un groupe alcoyle ayant de 1 à 4 atomes de carbone, trifluorométhyle, carboxyméthyle, hydroxy, amino, alcoylamino ayant de 1 à 4 atomes de carbone dans la partie alcoyle, dialcoylamino ayant de 1 à 4 atomes de carbone dans chaque partie alcoyle, phényle ou par un hétérocycle à 5 ou 6 chaînons qui peut contenir de 1 à 2 atomes d'azote et éventuellement également encore un atome de soufre ou d'oxygène; un groupe alcoyle ayant de 1 à 4 atomes de carbone ou un groupe acyle aliphatique ayant de 1 à 4 atomes de carbone, et dans laquelle le groupe méthoximino possède la position syn; ainsi que leurs sels et esters pharmaceutiquement acceptables.

2. Procédé pour la préparation de composés céphème de formule générale I, caractérisé en ce que;

a) on fait réagir des composés de céphème de formule générale II

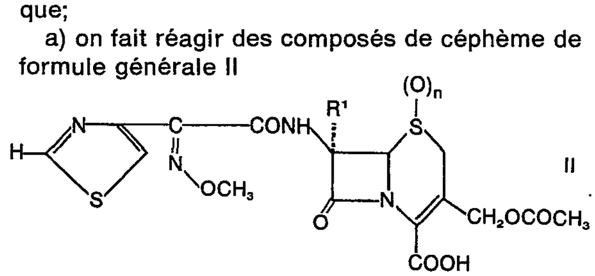

II

dans laquelle $R^1$ et n ont les significations indiquées ci-dessus, en présence d'un solvant et d'une base, avec un azide ou avec des composés de formule $HSR^3$ ou $R^3$ à la signification indiquée ci-dessus, ou

b) on fait réagir des composés de céphème de formule générale III sous la forme de leurs sels ou esters

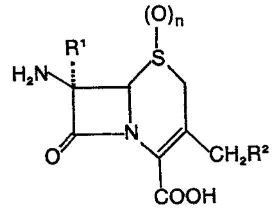

III

dans laquelle formule n, $R^1$ et $R^2$ ont les significations indiquées ci-dessus, avec des dérivés réactifs de l'acide 2-(1,3-thiazol-4-yl)-2-syn-méthoximino-acétique et dans les composés préparés selon a) ou b) si l'on désire:

α) on convertit un sel obtenu en l'acide carboxylique libre et on estérifie éventuellement ensuite celui-ci, ou

β) on convertit directement un sel obtenu en un ester, ou

γ) on saponifie un ester obtenu et on convertit éventuellement l'acide céphème carboxylique obtenu en un sel, ou

δ) on oxyde ultérieurement un composé de formule I obtenu avec n = 0 ou on réduit ultérieurement un composé de formule I avec n = 1, et si l'on désire, on convertit complémentairement les composés de céphème obtenus selon les stades α, β ou γ indiqués.

3. Procédé selon la revendication 2, caractérisé en ce qu'on utilise les composés de formule II et de formule $HSR^3$ sous la forme de leurs sels.

4. Compositions pharmaceutiques efficaces contre les infections bactériennes, caractérisées en ce qu'elles contiennent des dérivés de céphème de formule générale I.

**Revendications pour l'Etat contractant:** AT

1. Procédé pour la préparation de composés de céphème de formule générale I

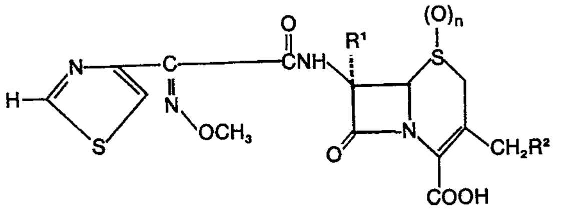

I

dans laquelle n est 0 ou 1, $R^1$ représente l'hydrogène ou un groupe alcoxy ayant de 1 à 4 atomes de carbone et $R^2$ représente un groupe azido ou $-SR^3$, où $R^3$ représente un hétérocycle à 5 chaînons ayant de 1 à 4 atomes d'azote et éventuellement un atome de soufre, un hétérocycle à 5 chaînons ayant de 1 à 2 atomes d'azote et un atome d'oxygène ou un hétérocycle à 6 chaînons ayant de 1 à 3 atomes d'azote et éventuellement un atome de soufre et où ces hétérocycles peuvent également être encore condensés sur un noyau benzène ou peuvent être substitués par un groupe alcoyle ayant de 1 à 4 atomes de carbone, trifluorométhyle, carboxyméthyle, hydroxy, amino, alcoylamino ayant de 1 à 4 atomes de carbone dans la partie alcoyle, dialcoylamino ayant de 1 à 4 atomes de carbone dans chaque partie alcoyle, phényle ou par un hétérocycle à 5 ou 6 chaînons qui peut contenir de 1 à 2 atomes d'azote et éventuellement également encore un atome de soufre ou d'oxygène; un groupe alcoyle ayant de 1 à 4 atomes de carbone ou un groupe acyle aliphatique ayant de 1 à 4 atomes de carbone, et dans laquelle le groupe méthoximino possède la position syn; ainsi que de leurs sels et esters pharmaceutiquement acceptables, lequel procédé est caractérisé en ce que:

a) on fait réagir des composés de céphème de formule générale II

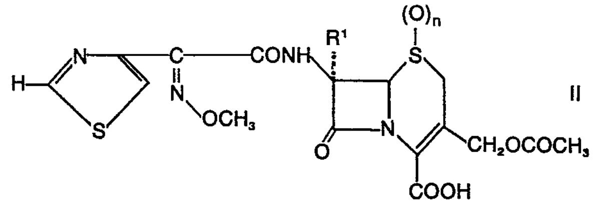

II

dans laquelle $R^1$ et n ont les significations indiquées ci-dessus, en présence d'un solvant et d'une base, avec un azide ou avec des composés de formule $HSR^3$, où $R^3$ a la signification indiquée ci-dessus, ou

b) on fait réagir des composés de céphème de formule générale III sous la forme de leurs sels ou esters

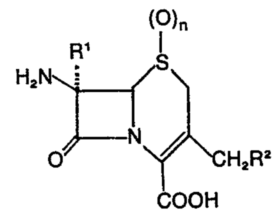

III

dans laquelle formule n, $R^1$ et $R^2$ ont les significations indiquées ci-dessus avec des dérivés réactifs de l'acide 2-(1,3-thiazol-4-yl)-2-syn-méthoximino-acétique et dans les composés préparés selon a) ou b) si l'on désire:

α) on convertit un sel obtenu en l'acide carboxylique libre et on estérifie éventuellement ensuite celui-ci, ou

β) on convertit directement un sel obtenu en un ester, ou

γ) on saponifie un ester obtenu et on convertit éventuellement l'acide céphème carboxylique obtenu en un sel, ou

δ) on oxyde ultérieurement un composé de formule I obtenu avec n = 0 ou on réduit ultérieurement un composé de formule I obtenu avec n = 1 et, si l'on désire, on convertit complémentairement les composés de céphème obtenus selon les stades α, β ou γ.

2. Procédé selon la revendication 1, caractérisé en ce qu'on utilise les composés de formule II et de formule $HSR^3$ sous la forme de leurs sels.

**Claims for the Contracting States:** BE, CH, DE, FR, GB, IT, LI, NL

1. Cephem compounds of the general formula I

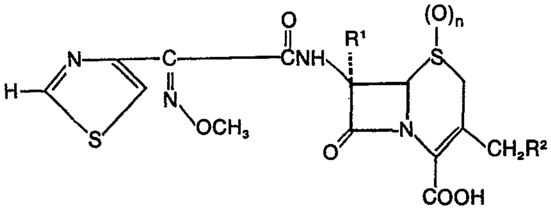

I

wherein n is 0 or 1, $R^1$ is hydrogen or alkoxy having 1 to 4 carbon atoms and $R^2$ is azido or a radical $-SR^3$, in which $R^3$ is a five-membered heterocycle with 1 to 4 nitrogen atoms and optionally one sulfur atom, a five-membered heterocycle with 1 to 2 nitrogen atoms and one oxygen atom or a six-membered heterocycle with 1 to 3 nitrogen atoms and optionally one sulfur atom, wherein these heterocycles may also carry a fused benzene nucleus or may be substituted by alkyl with 1 to 4 carbon atoms by trifluormethyl, by carboxymethyl, by hydroxy, by amino, by alkyl-amino with 1 to 4 carbon atoms in the alkyl part, by dialkyl-amino with 1 to 4 carbon atoms in each alkyl part, by phenyl or by a five- or six-membered heterocycle which may contain 1 to 2 nitrogen atoms and optionally also one sulfur or oxygen atom, alkyl with 1 to 4 carbon atoms or aliphatic acyl with 1 to 4 carbon atoms, and in which the methoximino group is in the syn-position, and pharmacologically acceptable salts and esters thereof.

2. Process for the manufacture of cephem compounds of the formula I which comprises

a) reacting cephem compounds of the general formula II

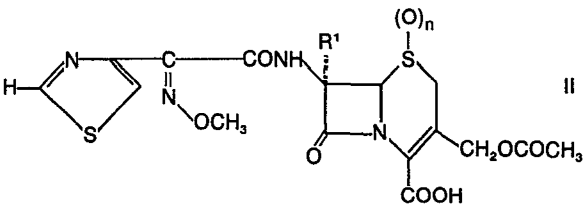

II

wherein $R^1$ and n are as defined above, in the presence of a solvent and a base, with an azide or with compounds of the formula $HSR^3$, in which $R^3$ is as defined above or,

b) reacting cephem compounds of the general formula III in the form of their salts or esters

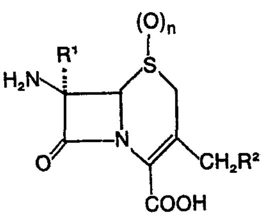

III

wherein n, $R^1$ and $R^2$ are as defined above, with reactive derivatives of 2-(1,3-thiazol-4-yl)-2-syn-methoximinoacetic acid and, if desired, in the compounds obtained according to a) or b)

α) converting a salt obtained into the free carboxylic acid and optionally further esterifying the latter or

β) converting a salt obtained directly into an ester or

γ) saponifying an ester obtained and optionally converting the carboxylic acid obtained into a salt or

δ) subsequently oxidizing a compound of the formula I wherein n is zero or subsequently reducing a compound of the formula I in which n is 1 and, if desired, further converting the resulting cephem compounds obtained according to steps α, β or γ.

3. The process as claimed in claim 2, which comprises using the compounds of the formula II and of the formula $HSR^3$ in the form of their salts.

4. Pharmaceutical preparations having an activity against bacterial infections characterized by a content of cephem derivatives of the formula I.

**Claims for the Contracting State:** AT

1. Process for the manufacture of cephem compounds of the general formula I

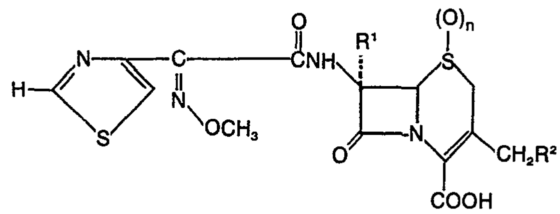

I

wherein n is 0 or 1, $R^1$ is hydrogen or alkoxy having 1 to 4 carbon atoms and $R^2$ is azido or a radical $-SR^3$, in which $R^3$ is a five-membered heterocycle with 1 to 4 nitrogen atoms and optionally one sulfur atom, a five-membered heterocycle with 1 to 2 nitrogen atoms and one oxygen atom or a six-membered heterocycle with 1 to 3 nitrogen atoms and optionally one sulfur atom, wherein these heterocycles may also carry a fused benzene nucleus or may be substituted by alkyl with 1 to 4 carbon atoms by trifluormethyl, by carboxymethyl, by hydroxy, by amino, by alkyl-amino with 1 to 4 carbon atoms in the alkyl part, by dialkyl-amino with 1 to 4 carbon atoms in each alkyl part, by phenyl or by a five- or six-membered heterocycle which may contain 1 to 2 nitrogen atoms and optionally also one sulfur or oxygen atom, alkyl with 1 to 4 carbon atoms or aliphatic acyl with 1 to 4 carbon atoms, and in which the methoximino group is in the syn-position, and pharmacologically acceptable salts and esters thereof, which comprises

a) reacting cephem compounds of the general formula II

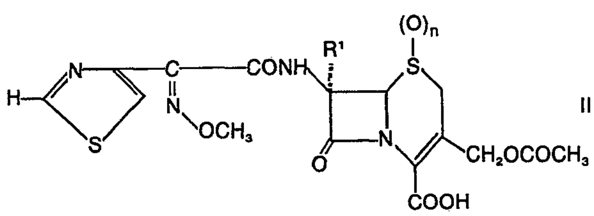

II

wherein $R^1$ and n are as defined above, in the presence of a solvent and a base, with an azide or with compounds of the formula $HSR^3$, in which $R^3$ is as defined above or,

b) reacting cephem compounds of the general formula III in the form of their salts or esters

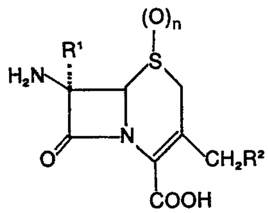

III

wherein n, $R^1$ and $R^2$ are as defined above, with reactive derivatives of 2-(1,3-thiazol-4-yl)-2-syn-methoximinoacetic acid and, if desired, in the compounds obtained according to a) or b)

α) converting a salt obtained into the free carboxylic acid and optionally further esterifying the latter or

β) converting a salt obtained directly into an ester or

γ) saponifying an ester obtained and optionally converting the carboxylic acid obtained into a salt or

δ) subsequently oxidizing a compound of the formula I wherein n is zero or subsequently reducing a compound of the formula I in which n is 1 and, if desired, further converting the resulting cephem compounds obtained according to steps α, β or γ.

2. The process as claimed in claim 1, which comprises using the compounds of the formula II and of the formula $HSR^3$ in the form of their salts.